# EUROPEAN PATENT SPECIFICATION

(11) **EP 0 755 910 B1**
(45) Date of publication and mention of the grant of the patent: **24.10.2001**
(21) Application number: 96111682.9
(22) Date of filing: 19.07.1996
(51) Int. Cl.: C07C 29/19, C07C 35/08

(54) **Process for preparing 4-tert.-butylcyclohexanol**
Verfahren zur Herstellung von 4-tert-Butylcyclohexanol
Procédé de préparation du 4-tert-butylcyclohexanol

(30) Priority: 20.07.1995 JP 18423895
(43) Date of publication of application: 29.01.1997
(73) Proprietor: Sumitomo Chemical Company, Limited, Chuo-ku Osaka 541-8550 (JP)
(72) Inventor: Sekiguchi, Masahito, Niihama-shi, Ehime-ken (JP); Tanaka, Shin, Niihama-shi, Ehime-ken (JP)
(74) Representative: VOSSIUS & PARTNER

(56) References cited:
- EP-A- 0 427 965
- CHEMICAL ABSTRACTS, vol. 119, no. 4, 26 July 1993 Columbus, Ohio, US; abstract no. 30360k, KONUSPAEV ET AL.: "Stereoselective hydrogenation of alkylphenols. I. ..." page 128; column 1; XP002033048 & KINET. KATAL., vol. 34, no. 1, 1993, pages 82-86,

## Description

The present invention relates to a process for preparing 4-tert.-butylcyclohexanol containing a larger amount of the cis-isomer by hydrogenating 4-tert.-butylphenol.

4-tert.-Butylcyclohexyl acetate is widely used as a perfume for cosmetics including soaps, and fragrance of its cis-isomer is more favorable than that of its trans-isomer. To prepare 4-tert.-butylcyclohexyl acetate having a high cis-isomer content, it is desired to provide a process for preparing 4-tert.-butylcyclohexanol containing its cis-isomer in a larger amount as a raw material of 4-tert.-butylcyclohexyl acetate.

In general, 4-tert.-butylcyclohexanol is prepared by hydrogenating 4-tert.-butylphenol.

JP-B-42-13938 discloses a process for preparing 4-tert.-butylcyclohexanol comprising catalytically reducing 4-tert.-butylphenol in the presence of a rhodium base catalyst.

MARUZEN OIL TECHNICAL REVIEW (MARUZEN SEKIYU GIHO) (1971) page 77 discloses a process for preparing 4-tert.-butylcyclohexanol comprising hydrogenating 4-tert.-butylphenol in the presence of various transition metals of the 8 to 10 Groups of the Periodic Table.

JP-A-54-122253 discloses a process for preparing a cis alkylcyclohexanol comprising hydrogenating an alkylphenol in the presence of a ruthenium-alumina catalyst.

US-A-2927127 discloses a process for preparing 4-tert.-butylcyclohexanol which has a high cis-isomer content comprising hydrogenating 4-tert.-butylphenol.

JP-A-3-173842 discloses a process for preparing 4-tert.-butylcyclohexanol comprising hydrogenating 4-tert.-butyl-phenol in the presence of a combined catalyst of Rh supported on a carrier and a boron fluoride type acid such as HBF₄.

EP-A-0 427 965 discloses a process for the preparation of 4-tert.-butylcyclohexanol, wherein the corresponding phenol is hydrogenated in the presence of a supported rhodium catalyst and HBF₄ or other stabilized BF₃.

Konnuspaev, S.R. et al., Kinetika I Kataliz, 1993, Vol. 34, No.1, pp. 82-86 reports on a study of the hydrogenation of p-tert.-butylphenol using different types of Rh-catalysts under various reaction conditions.

However, the cis-isomer content in 4-tert.-butylcyclohexanol which is prepared by the processes disclosed in JP-B-42-13938, MARUZEN OIL TECHNICAL REVIEW and JP-A-54-122253 is still insufficient. The process of US-A-2927127 achieves a high cis-isomer content in ethanol in the presence of the rhodium catalyst, but the reaction should be performed under a high hydrogen pressure. Then, an improvement has been sought as a preparation process of 4-tert.-butylcyclohexanol. Further, since the process of JP-A-3-173842 uses the boron fluoride type acid, a workload is required to recover fluorine and boron, and the generated acids such as HF corrode a production equipment.

An object of the present invention is to provide a process for preparing 4-tert.-butylcyclohexanol which can be carried out under a mild condition and produce 4-tert.-butylcyclohexanol having a high cis-isomer content.

This object has been achieved by a process for preparing 4-tert.-butylcyclohexanol comprising hydrogenating 4-tert.-butylphenol in a solvent in the presence of a rhodium catalyst and hydrogen chloride and/or (anhydrous) sulfuric acid.

The rhodium catalyst to be used in the hydrogenation reaction according to the present invention includes rhodium metal (valency of zero) or a compound of rhodium having a valency of up to 6 such as rhodium chloride, rhodium oxide and so on.

The rhodium metal or rhodium compound is used in the form of a supported type catalyst, that is, the rhodium metal or rhodium compound is supported on a carrier such as activated carbon, SiO₂, Al₂O₃, etc. Among the supported type catalyst, rhodium metal supported on the carrier is more preferred. In the case of the supported type catalyst, a supported amount of rhodium metal is usually from 1 to 10 wt. %, preferably 3 to 5 wt. % based on the weight of the carrier.

After the reaction, the rhodium catalyst may be recovered from a reaction mixture by a conventional method such as filtration, decantation, centrifugation, and recycled.

While an amount of the catalyst (including the carrier) depends on the supported amount of the rhodium metal or compound, it is from about 0.1 to 50 wt. % (in a dry form) based on the weight of 4-tert.-butylphenol. As the amount of the catalyst increases, the selectivity of the cis-isomer increases. The amount of the catalyst is preferably from 0.5 to 10 wt. % in view of the cost and workability in the filtration step for recovering the catalyst.

Any solvent may be used as long as it has no adverse effect on the reaction. A solvent which is liquid at room temperature (25°C) is preferred because of easy handling. Examples of the solvent are alkanes having 5 to 10 carbon atoms, ethers having 4 to 10 carbon atoms, alcohols having 1 to 6 carbon atoms, and so on. Specific examples of the solvent are acyclic alkanes (e.g. pentane, hexane, heptane, etc.), cyclic alkanes (e.g. cyclohexane, etc.), acyclic ethers (e.g. diethyl ether, etc.), cyclic ethers (e.g. tetrahydrofuran, dioxane, etc.), and alcohols (e.g. methanol, ethanol, propanol, isopropanol, butanol, isobutanol, pentanol, hexanol, 4-methyl-2-pentanol, cyclohexanol, etc.). Among them, cyclohexane and isopropanol are preferred. In particular, isopropanol is preferred.

The amount of the solvent is usually from about 0.2 to 20 times, preferably from 0.4 to 5 times the weight of 4-tert.-butylphenol.

In the process of the present invention, the reaction is performed in the solvent in the presence of the rhodium catalyst and also hydrogen chloride or (anhydrous) sulfuric acid.

Hydrogen chloride may be supplied in the reaction system in any form, for example, by bubbling hydrogen chloride gas through the reaction system, or adding hydrochloric acid to the reaction system. Alternatively, hydrochloric acid may be formed in the reaction system, for example, by charging water and AlCl₃ or TiCl₄ to the reaction system. In addition, a catalyst which generates hydrogen chloride in the reaction system such as rhodium chloride may be used.

Also, (anhydrous) sulfuric acid may be supplied in the reaction system in any form, for example, by bubbling SO₃ gas through the reaction system or adding an aqueous sulfuric acid solution to the reaction system.

The order of the addition of the raw material, the rhodium catalyst, the solvent and hydrogen chloride or (anhydrous) sulfuric acid is arbitrary.

The amount of hydrogen chloride or (anhydrous) sulfuric acid is from 0.1 to 10 moles per one mole of the rhodium atom in the rhodium catalyst.

The process of the present invention may be carried out in a stream of hydrogen gas or in a pressurized hydrogen atmosphere. Other reaction conditions may not be critical. In view of a reaction rate, the reaction is preferably carried out in the pressurized hydrogen atmosphere. In this case, a pressure reactor is used.

When the reaction is carried out under the pressurized hydrogen atmosphere, a partial pressure of hydrogen is at least about 1.5 x 10⁵ Pa. In view of the reaction rate, the selectivity of the cis-isomer and the pressure resistance of the equipment, the partial pressure of hydrogen is preferably from 3 x 10⁵ to 2 x 10⁶ Pa, more preferably from 5 x 10⁵ to 1.5 x 10⁶ Pa.

A reaction temperature is at least about 20°C in view of the reaction rate and the selectivity of the cis-isomer, and preferably 100°C or lower in view of the selectivity of the cis-isomer. More preferably, the reaction temperature is from 40 to 80°C in view of the reaction rate and the selectivity of the cis-isomer.

The process of the present invention may be carried out continuously or batchwise.

The termination of the reaction can be confirmed by a conventional method. For example, the reaction mixture is analyzed and a time when the conversion of 4-tert.-butylphenol is 100 % is used as the termination of the reaction, or a time when no further decrease of the hydrogen pressure is observed is used as the termination of the reaction.

The process of the present invention can easily prepare 4-tert.-butylcyclohexanol having the high content of the cis-isomer useful as a raw material of a perfume from 4-tert.-butylphenol. That is, 4-tert.-butylcyclohexanol can be obtained at a yield of about 90 % or higher, and the content of the cis-isomer in the product reaches about 80 % or more.

The present invention will be illustrated by the following Examples, which do not limit the present invention in any way.

### Example 1

In an autoclave, there were charged 4-tert.-butylphenol (90 g, 0.60 mole), 5 %Rh/C (namely 5 wt. % of rhodium metal supported on a activated carbon carrier) (1.35 g based on the dry material), isopropanol (180 g) and 36 % hydrochloric acid (0.18 g), and then the interior of the autoclave was replaced with nitrogen gas by injecting the nitrogen gas up to 5 x 10⁵ Pa and evacuating it three times. After replacing the interior of the autoclave with hydrogen by injecting the hydrogen gas up to 5 x 10⁵ Pa and evacuating it three times, the hydrogen gas was injected up to 1.1 x 10⁶ Pa, and an interior temperature was raised to 60°C, followed by stirring for 1.75 hours.

After cooling the autoclave and replacing the interior with the nitrogen gas in the same way as above, the reaction mixture was analyzed to find that the yield of 4-tert.-butylcyclohexanol was 93.4 %, and a ratio of the cis-isomer to trans-isomer was 89.9:10.1.

### Examples 2-10

In the same manner as in Example 1 except that the reaction conditions were changed as shown in the Table, 4-tert.-butylcyclohexanol was prepared. In Example 10, 98 % sulfuric acid was used.

The results are shown in the Table.

In all of Examples 1-10, the conversion of 4-tert.butylphenol was 100 %.

### Comparative Examples 1-3

In the same manner as in Example 1 except that no acid was used (Comparative Example 1), phosphoric acid (85 %) was used (Comparative Example 2) or nitric acid (61 %) was used (Comparative Example 3), 4-tert.-butylcyclohexanol was prepared.

The results are shown in the Table.

### Comparative Examples 4 and 5

In the same manner as in Example 1 except that a Ru catalyst (5 %Ru/C) was used (Comparative Examples 4 and 5) and no hydrochloric acid was used (Comparative Example 5), 4-tert.-butylcyclohexanol was prepared.

The results are shown in the Table.

In Comparative Example 4, the conversion of 4-tert.-butylphenol was 42.2 %, while in other Comparative Examples, the conversion of 4-tert.-butylphenol was 100 %.

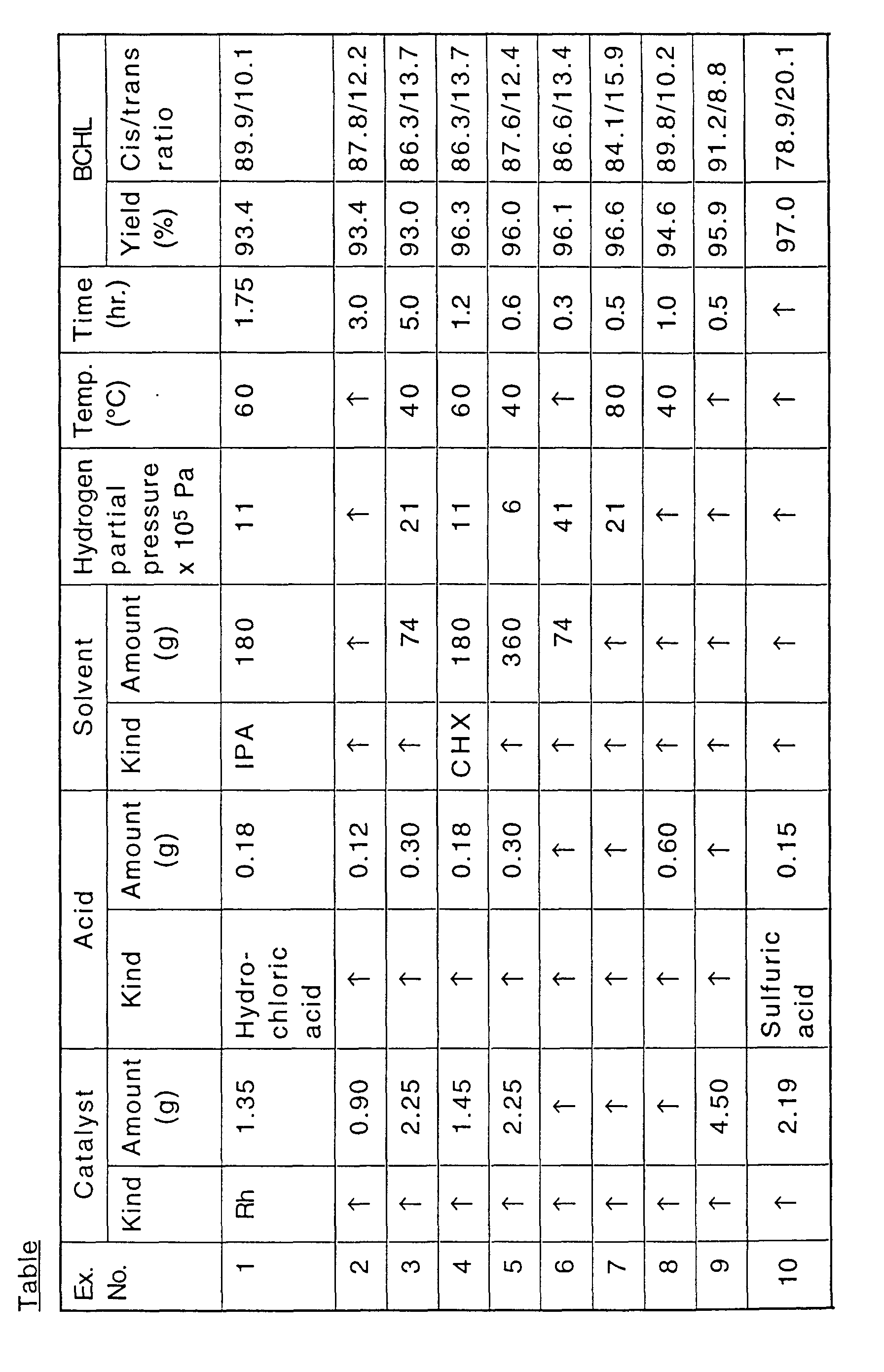

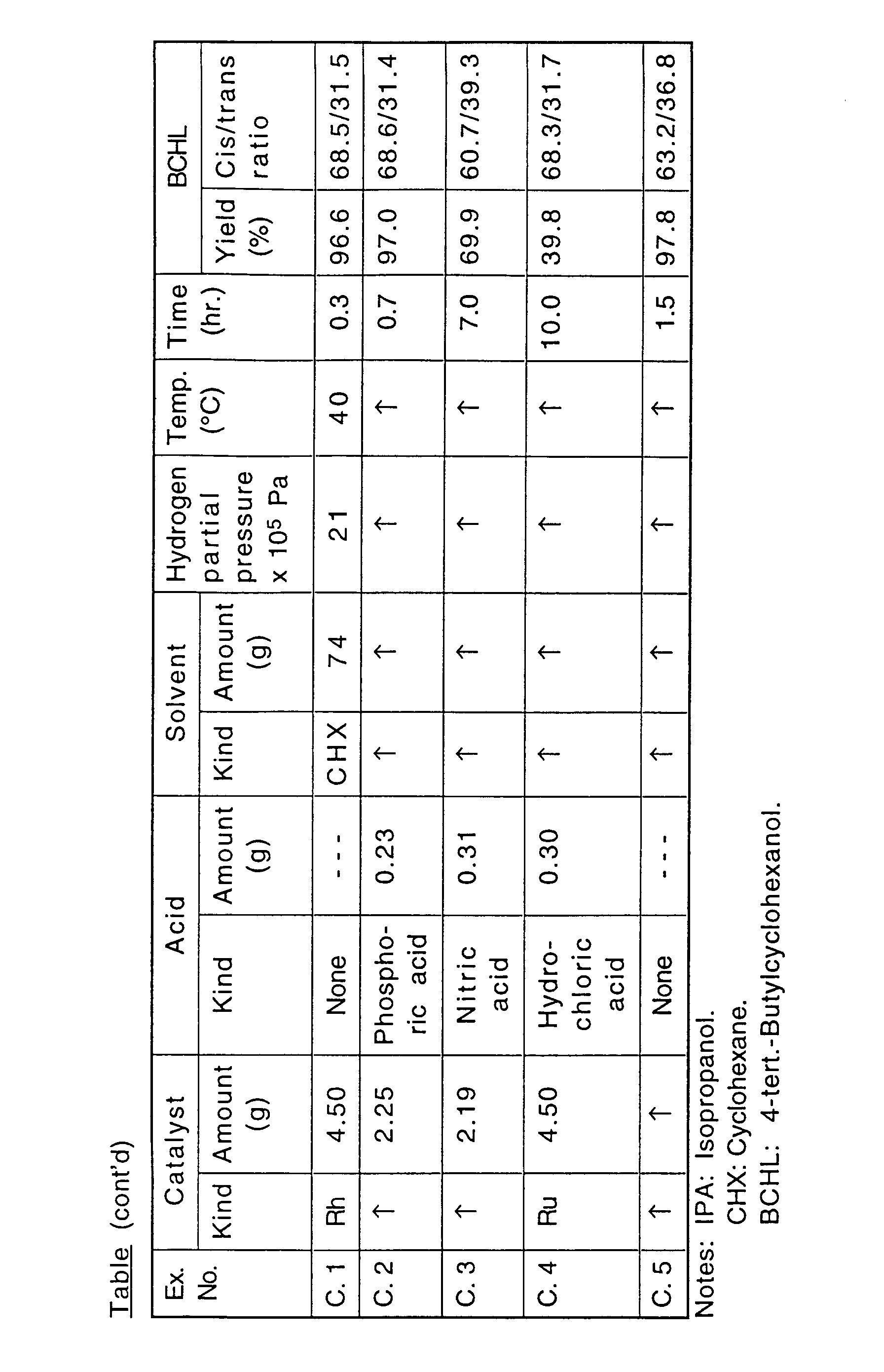

## Claims

1. A process for preparing 4 tert.-butylcyclohexanol comprising hydrogenating 4-tert.-butylphenol in a solvent in the presence of a rhodium catalyst and hydrogen chloride and/or anhydrous sulfuric acid or sulfuric acid, wherein the amount of the acid is from 0.1 to 10 moles per one mole of the rhodium atom in the rhodium catalyst and wherein said rhodium catalyst is a supported catalyst, selected from the group consisting of rhodium metal and rhodium compounds.

2. The process according to claim 1, wherein said rhodium catalyst comprises rhodium metal supported on a carrier.

3. The process according to claim 2, wherein an amount of the rhodium catalyst (in terms of the dry material)is from 0.5 to 10 wt. % based on the weight of 4-tert.-butylphenol.

4. The process according to any of claims 1 to 3, wherein said solvent is a solvent selected from alkanes having 5 to 10 carbon atoms, ethers having 4 to 10 carbon atoms and alcohols having 1 to 6 carbon atoms.

5. The process according to claim 4, wherein said solvent is an alcohol.

6. The process according to claim 5, wherein said alcohol is isopropanol.

7. The process according to any of claims 1 to 6, wherein a reaction temperature is from 20 to 100°C.

8. The process according to claim 1, wherein the amount of the acid is from 1.4 to 10 moles per mole of rhodium atoms in the rhodium catalyst.

## Patentansprüche

1. Verfahren zur Herstellung von 4-tert-Butylcyclohexanol, umfassend die Hydrierung von 4-tert-Butylphenol in einem Lösungsmittel in Gegenwart eines Rhodiumkatalysators und Salzsäure und/oder wasserfreier Schwefelsäure oder Schwefelsäure, wobei die Menge der Säure 0,1 bis 10 mol pro Mol Rhodiumatome im Rhodiumkatalysator beträgt und wobei der Rhodiumkatalysator ein auf einem Träger angebrachter Katalysator ist, ausgewählt aus Rhodiummetall und Rhodiumverbindungen.

2. Verfahren nach Anspruch 1, wobei der Rhodiumkatalysator auf einem Träger gehaltenes Rhodiummetall umfaßt.

3. Verfahren nach Anspruch 2, wobei eine Menge des Rhodiumkatalysators (hinsichtlich des Trockenmaterials) 0,5 bis 10 Gew.-% beträgt, bezogen auf das Gewicht von 4-tert-Butylphenol.

4. Verfahren nach einem der Ansprüche 1 bis 3, wobei das Lösungsmittel ein aus Alkanen mit 5 bis 10 Kohlenstoffatomen, Ethem mit 4 bis 10 Kohlenstoffatomen und Alkoholen mit 1 bis 6 Kohlenstoffatomen ausgewähltes Lösungsmittel ist.

5. Verfahren nach Anspruch 4, wobei das Lösungsmittel ein Alkohol ist.

6. Verfahren nach Anspruch 5, wobei der Alkohol Isopropanol ist.

7. Verfahren nach einem der Ansprüche 1 bis 6, wobei eine Reaktionstemperatur von 20 bis 100°C herrscht.

8. Verfahren nach Anspruch 1, wobei die Menge der Säure 1,4 bis 10 mol pro Mol Rhodiumatome im Rhodiumkatalysator beträgt.

## Revendications

1. Procédé pour la préparation de 4-tert.-butylcyclohexanol comprenant l'hydrogénation de 4-tert.-butylphénol dans un solvant en présence d'un catalyseur de rhodium et de chlorure d'hydrogène et/ou d'acide sulfurique anhydre ou d'acide sulfurique, dans lequel la quantité de l'acide est comprise entre 0,1 et 10 mol pour une mole de l'atome de rhodium dans le catalyseur de rhodium et dans lequel ledit catalyseur de rhodium est un catalyseur supporté choisi parmi du métal de rhodium et des composés du rhodium.

2. Procédé selon la revendication 1, dans lequel ledit catalyseur de rhodium comprend du métal de rhodium supporté sur un support.

3. Procédé selon la revendication 2, dans lequel une quantité du catalyseur de rhodium (en termes du matériau sec) est comprise entre 0,5 et 10 % en poids rapportés au poids de 4-tert.-butylphénol.

4. Procédé selon l'une quelconque des revendications 1 à 3, dans lequel ledit solvant est un solvant choisi parmi des alcanes ayant de 5 à 10 atomes de carbone, des éthers ayant de 4 à 10 atomes de carbone et des alcools ayant de 1 à 6 atomes de carbone.

5. Procédé selon la revendication 4, dans lequel ledit solvant est un alcool.

6. Procédé selon la revendication 5, dans lequel ledit alcool est l'isopropanol.

7. Procédé selon l'une quelconque des revendications 1 à 6, dans lequel une température de réaction est de 20 à 100°C.

8. Procédé selon la revendication 1, dans lequel la quantité de l'acide est de 1,4 à 10 mol par mole d'atomes de rhodium dans le catalyseur de rhodium.
